# EUROPEAN PATENT SPECIFICATION

(11) **EP 0 797 772 B1**
(45) Date of publication and mention of the grant of the patent: **09.10.2002**
(21) Application number: 95941098.6
(22) Date of filing: 15.12.1995
(51) Int. Cl.: G01N 27/64

(54) **METHOD AND DEVICE FOR MEASURING ANALYTES BY ION MOBILITY SPECTROMETRY**
VERFAHREN UND VORRICHTUNG ZUR MESSUNG VON ANALYTEN MITTELS IONENMOBILITÄTSSPEKTROMETRIE
PROCEDE ET DISPOSITIF POUR LA MESURE D'ANALYTES PAR SPECTROMETRIE DE MOBILITE IONIQUE

(30) Priority: 15.12.1994 FI 945916
(43) Date of publication of application: 01.10.1997
(73) Proprietor: ENVIRONICS OY, SF-50100 Mikkeli (FI)
(72) Inventor: KOTIAHO, Tapio, FIN-50600 Mikkeli (FI); PAAKKANEN, Heikki, FIN-70110 Kuopio (FI)
(74) Representative: Brax, Matti Juhani
(86) International application number: FI9500684
(87) International publication number: WO96018893

(56) References cited:
- WO-A-90/09583
- WO-A-92/07255

## Description

The present invention relates to a method and an instrument for measurement of analytes by ion mobility spectrometry, in e.g. a fermentation reaction. The objective of the invention is especially a direct continuous on-line measurement of alcohol for control of the alcoholic fermentation, in e.g. beer and yeast fermentation.

Ion mobility spectrometry (IMS) is an analytical method in which the analyte molecules are ionized by ion/molecule reactions in a radioactive ion source. The subsequent ions are separated according to their different mobilities in a weak electrical field at atmospheric pressure. The ion mobility spectrometry has so far been used for many applications, such as detection of chemical warfare agents and drugs and for chromatographic applications. WO-A-9009583 describes a membrane inlet ion mobility spectrometer for detecting analyte molecules in a gas, e.g. in ambient air.

The use of ion mobility spectrometry has been restricted by its relatively low specificity and limited quantitative capability. This concerns especially the time-of-flight method in on-line measurement of analytes.

For the measurement of ethanol has previously been used e.g. gas chromatography, enzymatic methods, membrane inlet mass spectrometry and electrochemical and solid-state sensors. Many of these methods have, however, drawbacks, such as long analysis times in the chromatographic methods, limited life times of the enzymatic sensors, relatively high cost of mass spectrometers and low specificity and slow response of the solid state sensors.

The sterility requirements involved in bioprocesses hamper or prevent the sampling during the process. This is e.g. the case in penicillin manufacture, in which fenox acetic acid is used as the reactant for penicillin. It is essential for the process that the fenox acetic acid concentration is within certain limits. The determination of the fenox acetic acid concentration during the almost two week long fermentation is vital for the yield.

The solution according to the invention provides a considerable improvement of the above mentioned disadvantages. The invention is characterized in what is presented in the claims.

The objective of the invention is to develop a method and an instrument for the measurement of analytes contained in a sample solution, especially for direct continuous on-line measurement of analytes for process control, which method and instrument are simple, reliable and inexpensive. The objective of the invention is especially to develop a method and an instrument for measurement of alcohols, such as ethanol, and especially for on-line measurement of ethanol by ion mobility spectrometry, preferably by ion mobility spectrometry based on the aspiration method for monitoring fermentation reactions, such as alcoholic and yeast fermentations. The method and instrument according to the invention provide the industry with a fast and inexpensive process control. The instrument according to the invention produces easily and quickly an analysis of several samples and eliminates the separate handling of samples when measuring.

The embodiment of the invention offers a quite new and feasible method of measuring analytes with an aspiration-type ion mobility spectrometer. When only one membrane is needed, the gas content does not fall in difficult conditions below the observation limit, which has been the problem when using several membranes. Sampling of the analytes in bioprocesses increases the risk for contamination, and the whole process to be analyzed might even have to be interrupted when taking a sample. Use of a membrane reduces this risk. A continuous on-line measuring method is thus highly desired and requested for bioprocesses and is now embodied with the method and instrument according to the invention.

The method and instrument according to the invention are below described in detail with reference to the enclosed pictures 1-9, in which:
Fig. 1 is a schematic diagram of the instrument according to the invention installed for measurement of the fermentation process analyte.
Fig. 2a is a picture of the bilobate membrane inlet.

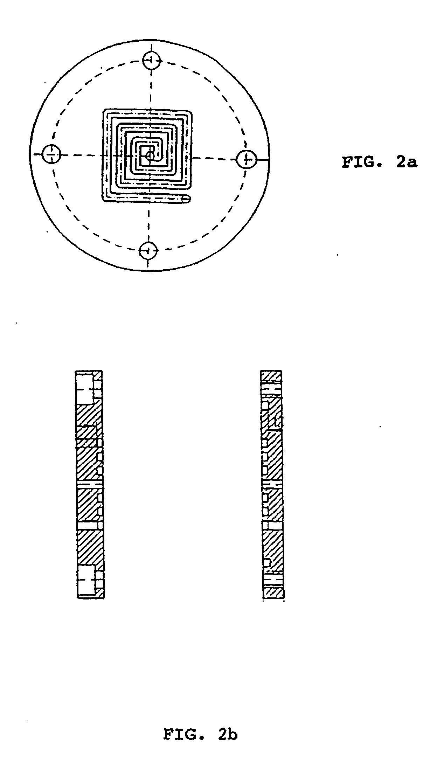

Fig 2 b is a cross-section of the Fig. 2a plates.

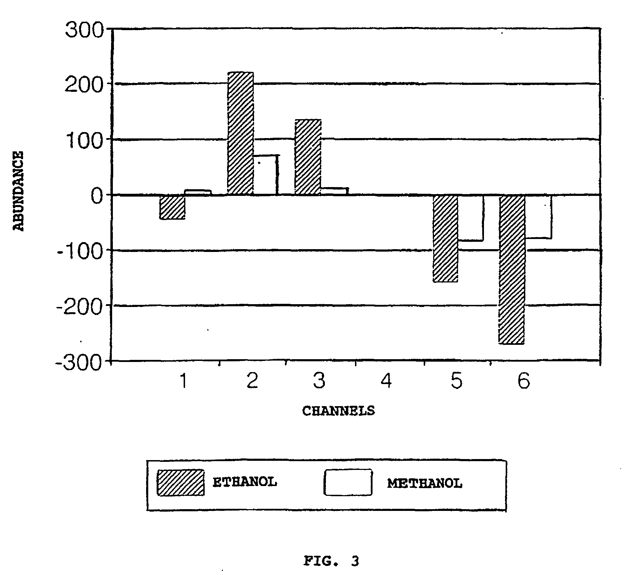

Fig. 3 presents the signal pattern, embodied by the instrument according to the invention for 2,5% (v/v) ethanol and methanol.

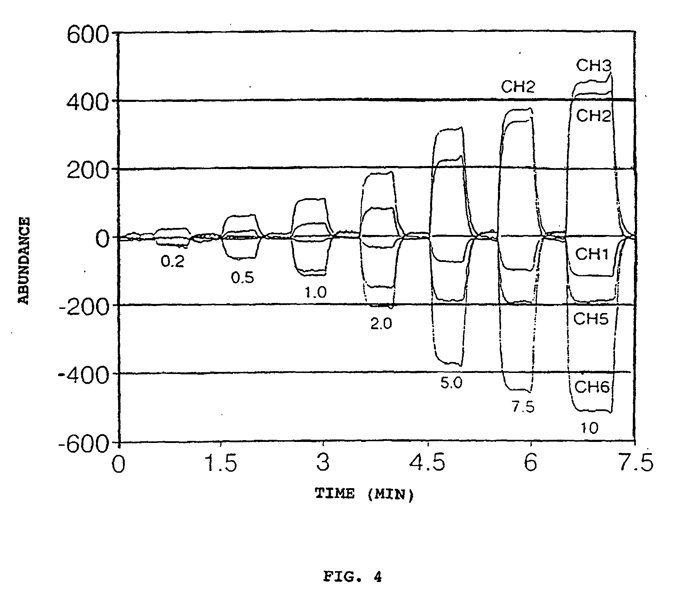

Fig. 4 shows signals of different channels for aqueous solutions of ethanol obtained by the instrument according to the invention.

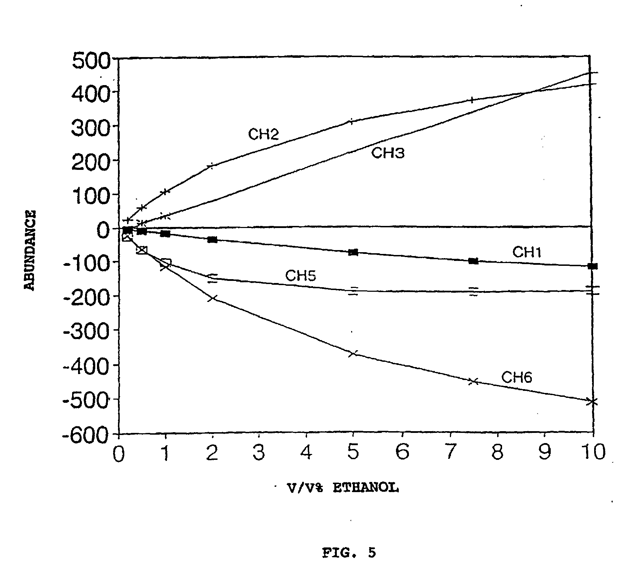

Fig. 5 presents ethanol calibration curves provided by the instrument according to the invention and extracted from the data presented in Fig. 4.

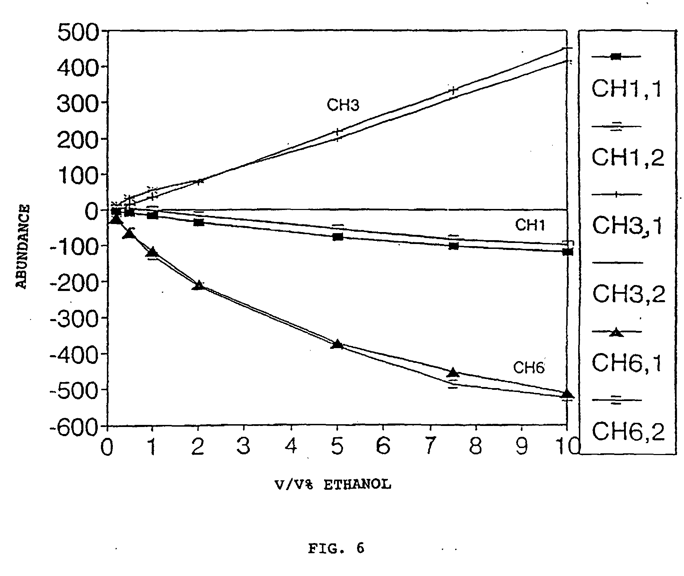

Fig. 6 presents ethanol calibration curves measured at two different days.

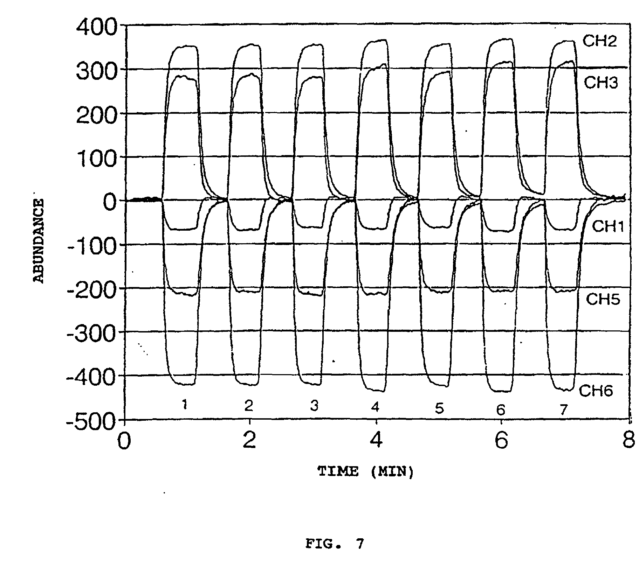

Fig. 7 shows signal patterns of different measurement channels for seven different beer brands.

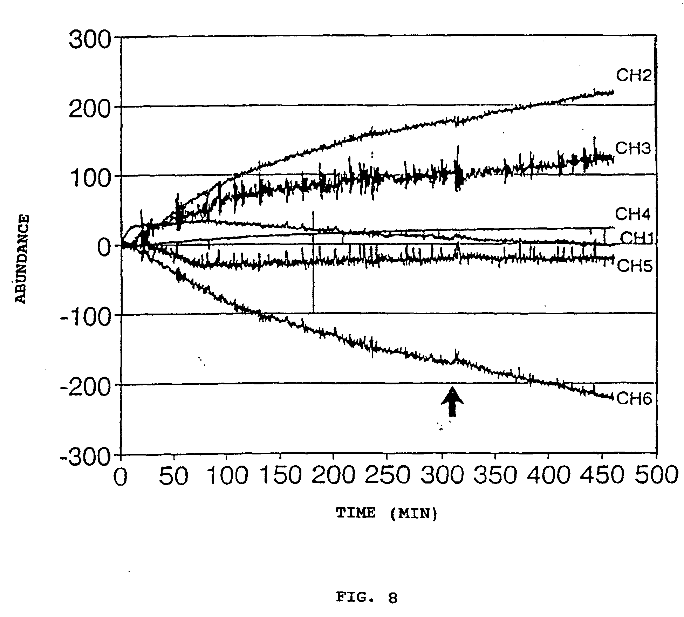

Fig. 8 shows signal patterns received from different detecting channels during yeast fermentation.

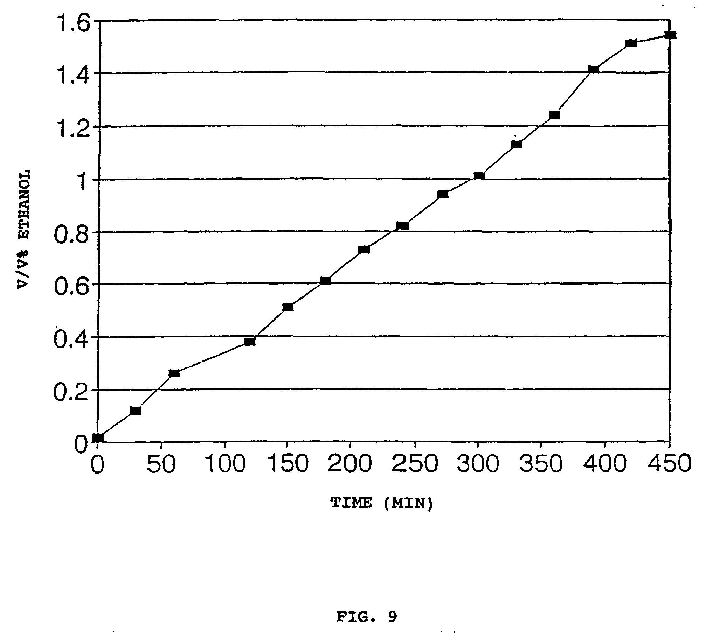

Fig. 9 presents ethanol concentration measurements of Fig. 8 yeast fermentation by membrane mass spectrometry.

The M90-gas detector 1 is an aspiration condenser-type ion mobility spectrometer, in which the air to be analyzed is led through a measuring cell. This device has more in detail been described in the Finnish patent 75055.

The M90-gas detector sensor comprises ionization, deflecting and measuring (collecting) regions. M90 uses a radiation source, e.g. a 160µCi ²⁴¹Am radioactive ion source for sample ionization. An important feature of the M90-instrument is that it contains separate deflecting and measuring regions for both positive and negative ions, which means that the gas flow can be divided into two separate flows, one for the measurement of positive ions and the other one for the measurement of negative ions. Electrical fields of a predetermined size transverse to the gas flow are formed in the deflecting and measuring regions with voltage plates. These regions are further divided into three different areas, which enables the simultaneous measurement of positive and negative ions at e.g. six different points, which secures the reliability of the result. Positive ions are measured in their own deflecting and measuring regions and negative ions in their own. Gas to be analyzed is aspirated into a tube, filtered with a heatable filter and led into the ionization cell, which as such can be provided several in parallel or in sequence. The gas is charged by radiation transmitted from e.g. the alpha- or beta-radiation source. The gas is led to a measuring tube. The voltage of the field electrodes in the collecting field is V₁, V₂,....Vₙ. The back plate voltage is V_{T}. In the collecting field the light ions charged in the gas are collected into the field electrodes Vₙ. In the measuring chamber the further advanced remaining heavy ions cause an ion current Iₙ to the electrodes in the chamber border, which is registered. From each value Iₙ, in which n is an integer, e.g. 1-6, is formed a diagram, the form of which depicts the substance to be analyzed.

During operation, ambient air and air containing gas to be measured are continuously pumped through the ionization, deflecting and measuring regions. After ionization of the sample air, part of the ions are collected to the deflection region and part of them are deflected to the measuring electrodes for measurement of the ion current by observing with an electrometer the current caused by the substance to be measured and comparing it with the calibration sample. Due to the continuous operation, the ambient air causes an even background signal level. Analytes in the sample air cause change in the ion population and therefore a positive or negative change compared to the background signal level is detected, and this change is the sample response.

The M90-instrument can be used alone or it can be connected to a personal computer 10, using a computer program for controlling the device. This program can be used for measurements and to change parameters. The most important parameters which can be changed using this program are collecting electrode gains and flow rate through the instrument. In addition, the program can be used to teach the instrument new compounds, i.e. to measure the characteristic signal pattern for a particular analyte and add the pattern to the library. During this so called standard measurement protocol, 2 signal values per second for each channel are recorded and stored in the computer.

The M90-detector type and the continuous signal measurement make it especially suitable for use in the method according to the invention, compared with other ion mobility spectrometers on market, compared e.g. with ion mobility spectrometers provided with a time-of-flight tube, in which the ion mobility is measured against the flow in a reverse electrical field while the current is changing in relation to time.

The building of a suitable interface between the measuring device and the component to be measured in a bioprocess is mainly determined by the nature of the process. In an aerobic process, the medium is bubbled with some proper gas, e.g. synthetic air or nitrogen, typically in the proportion 1:1 (v/v) to the volume of the fermentation chamber. Measuring the component evaporating from the medium from the headspace gas produces information about the component content in the medium itself. The amount of component in the headspace-gas can be affected with a proper mixture. In an anaerobic process, where the medium is not bubbled, the interface must be based on a membrane, whereby the component measured from the medium evaporates through a proper membrane. Temperature of the medium is typically 30 - 40 °C, causing the high relative humidity of the sample gas to create a problem in the first alternative. If determination of the reactant / end product is difficult due to the properties of the measuring device, the state of the process can also be measured indirectly. The bacteria or yeast metabolism yields several other gases or vapors that can be measured. One example is again the fenox acetic acid, which evaporates into the headspace-gas, but seems to contaminate immediately on tubes and measuring chambers, thus complicating the on-line measurement.

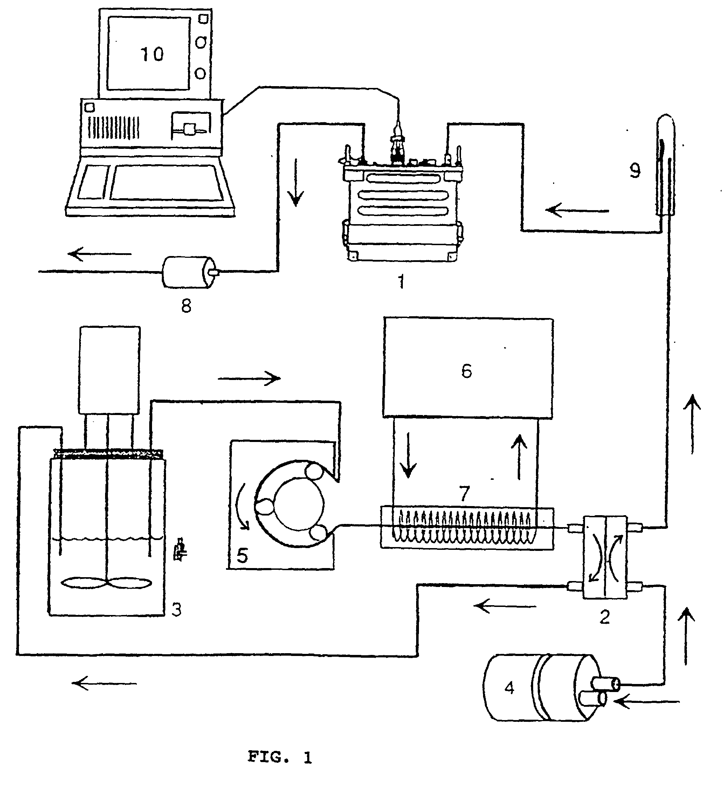

In the method according to the invention, the sample is extracted directly from the reaction vessel 3 as shown in Fig. 1, and is pumped with the sample pump 5 to the heat exchanger 7, which is heated with e.g. a water bath 6. From this the sample is led to the membrane inlet 2, which preferably is made of stainless steel. The membrane inlet comprises two plates opposite each other, with spiral grooves 20 provided in the areas coming opposite each other, along which grooves the sample can pass. The width of the grooves is in the example approx. 2 mm and the depth 1 mm (Figs. 2a and 2b). A microporous polypropylene membrane (Celgar 2502, Hoechst Celenase, North-Carolina, USA) is clamped between the plates, and an 0-ring is mounted to one of the sides. The Celgard 2502 membrane is 50 µm thick, and has an effective pore size of 0,075 µm and a porosity of 45%. The sample solution is circulated on one side of the membrane and air on the other side. The area of the membrane exposed to the sample solution and to the air is 3 cm². The evaporated analyte contained in the sample solution, which analyte is in a molecule form, passes through the membrane due to diffusion and laminar/turbulent flow interaction (F.R. Lauritsen and D. Lloyd, C. Fenselau (Ed.), "Mass Spectrometry for the Characterization of Microorganisms", ACS Symposium Series 541, American Chemical Society, Washington DC 1994, p. 91). The temperature of both the membrane inlet 2 and the input sample flow is properly regulated for the measuring device. The membrane inlet is surrounded by an insulating material in order to achieve a stable temperature.

As shown, the sample solution is hereafter conducted to one side of the membrane inlet described above, thus transferring the analyte to be measured through the membrane and with the assistance of the air pump 4 to the air stream flowing on the other side of the membrane. The air stream is then led through the water trap 9 to the ion mobility spectrometer. The water trap prevents the humidity from entering the M90-instrument.

### The experimental part

The operation of the subject method and instrument in monitoring fermentation processes was tested by measuring ethanol concentrations in yeast fermentation. The results of the yeast fermentation were compared with results measured by membrane inlet mass spectrometry.

The described ion mobility spectrometer and membrane inlet as well as other instrument parts were used in the tests. Balzers QMG 420 mass spectrometer was used as membrane inlet mass spectrometer. The special characteristics related to the use of this device have been described more in detail in the publication F.R. Lauritsen, L.T. Nielsen, H. Degn, D. Lloyd and S. Bohatka, Biol. Mass Spectrom. 20(1991)253. The temperature of the sample cell was 25 °C. The concentration of ethanol in yeast fermentation samples was measured using single ion monitoring (ion m/z 31 monitor) and external standard calibration. The sampling frequency was 2 samples per hour.

### Instrumental parameter determination

Standard solutions were made adding ethanol (96% (v/v)) or methanol to distilled water. Several beer brands were acquired from store.

Bakers yeast fermentations were carried out in a 2 liter fermentor at 33°C. Agitation was 400 rpm and working volume of the fermentation broth was 1,6 liters. The initial glucose concentration (D(+)-glucose monohydrate) and the bakers yeast concentration were 62,5 g/l and 12,5 g/l respectively. The fermentation medium was distilled water into which 1 g/1,6 l commercial wine/beer fermentation salt mixture (Vinicole A/S, Denmark) was added.

Deflection voltages were determined for ethanol by changing the deflection voltage default values of the M90-gas detector. The deflection voltages can be changed in the range of - 5 - + 5 V using the potentiometers connected to the sensor part of the M90 instrument. Continuous ethanol standard introduction via the sensor provided with a membrane was used during the calibration. The results of the calibration are shown in Fig. 3, which presents a signal pattern for the ethanol 2,5%-solution (v/v). The channels 1, 2 and 3 are for positive ions and the channels 4, 5 and 6 are for negative ions. As it can be seen from Fig. 3, there is a clear maximum of the signal at one positive ion channel and one negative ion channel. Fig. 3 displays also a signal pattern for the methanol 2,5%-solution (v/v). When comparing the ethanol and methanol signal patterns, it can be established that the M90 IMS-device can easily separate these two fairly similar chemical substances. It should be noted that the sensitivity of methanol is considerably lower than that of ethanol.

The normal air flow rate through the detector part of the instrument was 2,4 1/min. The effects of the membrane area, of the sample/membrane temperature and of the sample flow rate on the ethanol signal were also studied.

All measurements were implemented by using the above mentioned microporous polypropylene membrane, which was selected based on results obtained in the membrane mass spectrometry when analyzing small polar compounds direct from an aqueous sample, F.R. Lauritsen, T.K. Choudhury, L.E. Dejarme and R.G. Cooks, Anal. Chim. Acta, 266 (1992) 1.

The effect of membrane area on the ethanol signal was tested by measuring the ethanol signal of 5 different aqueous ethanol solutions (in the range 0,2 - 5% (v/v)), using two membrane inlets having different active membrane areas, i.e. 1,6 cm² and 3 cm². As expected, the measurements evidenced that a better sensitivity was achieved when using a large active membrane area. A 50% increase of the total signal level was achieved with a larger membrane area than by a smaller membrane area.

The large membrane area was used in the subsequent tests because of its better sensitivity.

The effect of the sample/membrane temperature on ethanol signal levels was tested by measuring at three different temperatures (25°C, 35°C and 45°C) using five different ethanol solutions (in the range of 0,2 - 5% (v/v). It was established that higher temperatures give better sensitivity. Increasing the sample/membrane temperature from 25°C to 45°C, increased the total signal levels about 50%. However, the use of higher temperatures is restricted by the higher moisture content of the sample air flow. The relative humidity of the air flow increased from 35% to 67% when the temperature was increased from 25°C to 45°C. The temperature 40°C was selected for all tests to obtain a good sensitivity.

The effect of the sample flow rate on the ethanol signal was tested using several sample flow rates in the range of 4,5 - 48 ml/min. No significant differences in the ethanol signal level were observed. A typical value for sample flow rate was 20 ml/min, and this was used in all measurements. The air flow rate through the membrane inlet was 1,4 l/min. in all measurements. Note that the air pumped through the membrane inlet and the air sucked by the M90 instrument via the water trap was unpurified laboratory air.

### Results of the ethanol measurements

Fig. 4 shows a typical response of various detector channels to the M90-ion mobility spectrometer calibrated as presented above for aqueous solutions of ethanol at 0,2, 0,5, 1,0, 2,0, 5,0, 7,5 and 10% (v/v) levels, as a function of time. Data in Fig. 4 were obtained by sequentially injecting 30-s injections of ascending ethanol concentrations into the continuous water stream passing through the membrane inlet. Channel 4 signal is not presented in Fig. 4, since it stayed at the constant background level throughout the whole experiment. The very good stability of the signals presented in Fig. 4 for each of the sample solutions illustrates the quantitative reproducibility of the membrane inlet ion mobility instrument.

From Fig. 4 it can be seen that the ethanol response is very fast. Rise times (10-90%) and fall times (90-10%) for various ethanol solutions were in the range of 5-10 s. Typically the rise times were a few seconds shorter than the fall times. The calibration curves presented in Fig. 5 were extracted from the data shown in Fig. 4. As can be seen from Fig. 5 signal linearity at channels 1 and 3 is relatively good in the whole concentration range, but for the other channels the signal is linear as a function of the ethanol concentration only in very small concentration ranges. Correlation coefficients of 0.989 and 0.999 were calculated for channel 1 and channel 3, respectively. It appears that the signals of the channels 1 and 3 are preferable for quantitative analysis when external standard calibration is used. However, good signal linearity is not necessarily required for good quantization, since nonlinear calibration curves can be also used for quantization, if the reproducibility of the calibration curves is good. In Fig. 6 calibration curves for channels 1, 3 and 6 measured during two different days are shown, indicating that calibration curves can be relatively well repeated even when the measurement system has been turned completely off and new standard solutions are being prepared for the second measuring. Typically the signal levels could be repeated within ± 10% on a day to day basis.

### Ethanol determination in commercial beers

Fig. 7 shows response of various measurement channels of the M90-instrument for seven different Danish beers. The alcohol content of the beers declared on the labels was 4,6% (v/v). Again the channel 4 signal is not presented since it stayed at the constant background level during the measurement. From Fig. 7 it can be seen that all the beer samples give responses of about the same magnitude, which is a good indication that the M90 IMS instrument can be used for quantitative ethanol measurements. Another indication of quantitative capabilities of membrane inlet ion mobility spectrometry is good reproducibility of signal levels, especially at the channels which give the best response for ethanol. Six times repeated measurements of the beer sample showed that a coefficient of variation value of 1% was obtained for channels 2, 5 and 6 and values of 6 and 10% for channels 3 and 1, respectively.

Ethanol concentrations of the beer samples were calculated based on two different external standard calibrations, one obtained using ethanol standards prepared in distilled water and the other obtained using ethanol standards prepared by adding ethanol to a light beer (original ethanol concentration 2,6% (v/v), ethanol added to get 5,0 and 7,5% (v/v) solutions). Ethanol concentrations shown in Table 1 were obtained by calculating the average for the 35 points on the height of the sample peak, and by using this average response value to determine the ethanol concentration from the calibration curves obtain as a result of standard measurements. The channel 5 was not used in these calculations since it was very close to the saturation level. The Table 1 results confirm the result observable from Fig. 7, in which the samples 4, 6 and 7 contain more ethanol than the other samples. Ethanol standards prepared in water gave too high alcohol contents from the channel 2, 3 and 6 signals. Alcohol contents of the beer relatively close to the declared contents were obtained only at channel 1. Standard solutions prepared in beer gave good quantitative results at all channels.

**Table 1**

| Beer sample | Channel 1 | | Channel 2 | | Channel 3 | | Channel 6 | |
|---|---|---|---|---|---|---|---|---|
| | Beer STD | Water STD | Beer STD | Water STD | Beer STD | Water STD | Beer STD | Water STD |
| 1 | 4.8 | 4.4 | 4.8 | 6.6 | 4.3 | 6.3 | 4.5 | 6.4 |
| 2 | 4.9 | 4.5 | 4.8 | 6.7 | 4.3 | 6.4 | 4.6 | 6.4 |
| 3 | 4.5 | 4.1 | 4.8 | 6.7 | 4.2 | 6.3 | 4.5 | 6.4 |
| 4 | 4.8 | 4.4 | 5.1 | 7.0 | 4.6 | 6.7 | 4.8 | 6.9 |
| 5 | 4.5 | 4.1 | 4.8 | 6.7 | 4.4 | 6.5 | 4.6 | 6.4 |
| 6 | 5.1 | 4.7 | 5.2 | 7.1 | 4.7 | 7.0 | 4.9 | 6.9 |
| 7 | 4.8 | 4.5 | 5.0 | 6.9 | 4.7 | 7.0 | 4.8 | 6.8 |

Table 2 presents results of other beer alcohol content measurements using the method developed above. This test was using beer samples with an alcohol content of 4,6 (v/v) (content declared on the bottle), as calibration standard solution. The choice of beer as standard solution is based on the results presented above, which disclosed that when determining the alcohol content of beer, the determination of the calibration curve using ethanol aqueous solutions give wrong results. From Table 2 it can be seen that the calibration method used gives relatively good results for the lower alcohol content beers, especially when channels 1 and 3 are considered. This result is understandable since the best linearity was observed at channels 1 and 3. The result for the high alcohol content beer is not very good which fact again confirms that the best quantization results will be obtained with a standard which is as similar as possible to the sample solution. Finally it should be noted that there generally are big variations in the alcohol contents declared for the commercial beers (4-5%).

**Table 2**

| **Beer sample** | **Channel 1** | **Channel 2** | **Channel 3** | **Channel 6** |
|---|---|---|---|---|
| 1, 2.6 v/v % | 2.6 | 3.4 | 2.6 | 3.3 |
| 2, 5.9 v/v % | 5.4 | 4.9 | 5.1 | 4.9 |
| 3, 9.4 v/v% | 7.8 | 6.1 | 8.9 | 5.2 |

### Ethanol determination in yeast fermentation

Ethanol production in yeast fermentation was also studied by on-line monitoring of the ethanol concentration by a membrane inlet ion mobility spectrometry. The measuring results are presented in Fig. 8, where the second adding of glucose is marked by arrow. The results in Fig. 8 display that the channel 2 and 6 signals show the expected result, i.e. a relatively constant increase of the signal as the yeast is growing and producing ethanol. The channels 1, 4 and 5 show, however, unexpected results. The channel 1 signal increases at first quickly, is then stabilized for a moment, whereafter it starts decreasing, as could be expected. Channel 4 indicates an even unexpected increase during the whole test and the channel 5 signal is saturated unexpectedly fast. It is, however, sufficient for the monitoring of the yeast fermentation that the channels 2, 3 and 6 give good results of the ethanol concentration growth. Ethanol concentration growth was also measured by membrane mass spectrometry, and the results are presented in Fig. 9. The results in Fig. 9 confirm the relatively even growth of ethanol concentration indicated by channels 2, 3 and 6. In the Fig. 8 test, glucose (50 g) was added 310 minutes after the starting of the fermentation (position of the arrow in Fig. 8), but no change in ethanol concentration *was* observed by either spectrometry (Figs. 8 and 9).

The method and instrument according to the invention can naturally be modified by an experienced craftsman within the scope of protection presented in the enclosed claims.

## Claims

1. A method for determining, by means of ion mobility spectrometry, the analyte content of a manufacturing process sample, comprising the steps of:
extracting the analyte from the sample by means of a gas in order to form a sample gas which contains the analyte;
ionizing the sample gas;
conducting the ionized sample gas through a measuring tube comprising electrodes for generating collecting electrical fields essentially vertical to the direction of the sample gas movement;
measuring the electrical current generated by the ions collected in said electrodes, said current being a measure of the analyte content;
**characterized by**
conducting a sample as a sample solution from the manufacturing process to a membrane inlet and along one side of the membrane; and
conducting a stream of the carrier gas to said membrane inlet and along the other side of the membrane,
whereby the analyte passes from the sample solution, due to diffusion and laminar/turbulent flow interaction, through the membrane into the carrier gas to form said sample gas which contains the analyte.

2. A method according to claim 1, **characterized in that** the carrier gas is air.

3. A method according to claim 1 or 2 **characterized in that** the analyte content is derived by comparing the electrical current caused at the single electrodes by the sample to be measured and by a calibration sample, respectively.

4. A method according to claim 1, 2 or 3, **characterized in that** the collection of ions having different mobility in different electrodes is measured.

5. A method according to any one of the preceding claims, **characterized in that** the temperature of the sample solution is regulated.

6. A method according to any one of the preceding claims, **characterized in that** the measurement of the collected analyte ions is carried out continuously on-line.

7. A method according to any one of the preceding claims, **characterized in that** the sample solution is pumped from the manufacturing process via a heat exchanger to the membrane inlet.

8. A method according to any one of the preceding claims, **characterized in that** the sample gas is led through a water trap before it is ionized for ion mobility spectrometry.

9. A method according to any one of the preceding claims, **characterized in that** the flow of the sample gas to the ion mobility spectrometer is 1 - 3 1/min., preferably 2 - 2.5 1/min.

10. A method according to any one of claims 1 to 7 and 9, **characterized in that** the sample gas is led directly to the ion mobility spectrometer.

11. A method according to any one of the preceding claims, **characterized in that** the process is a bioprocess, preferably a fermentation reaction, a yeast or bacteria fermentation, and/or that the analyte is ethanol or another volatile bioprocess product.

12. A method according to any one of the preceding claims, **characterized in that** the ionized sample gas is divided into two essentially similar flows, from which the content of the analyte is measured.

13. An instrument for measuring an analyte content of a sample of a manufacturing process, comprising a reaction vessel (3) and an ion mobility spectrometer (1) for measuring the analyte content, **characterized in that** it further comprises a liquid sample pump (5), a membrane device (2) having a membrane allowing the permeation of analyte molecules from a liquid to a gas phase, and a gas pump (4), which are arranged so that the liquid sample pump (5) pumps the sample as a sample solution from the vessel (3) to one side of the membrane, the gas pump (4) pumps carrier gas to the other side of the membrane, whereby the analyte passes from the sample solution, due to diffusion and laminar/turbulent flow interaction, through the membrane into the carrier gas to form a sample gas containing the analyte, and further pumps the sample gas to the ion mobility spectrometer (1).

14. An instrument according to claim 13, **characterized in that** the carrier gas is air.

15. An instrument according to claim 13 or 14, **characterized in that** a heat exchanger (7) is arranged between the liquid sample pump (5) and the membrane device (2) to heat the sample solution before it reaches the membrane.

16. An instrument according to claim 12, 13 or 14, **characterized in that** the membrane device (2) is a bilobate membrane device, comprising discoid stainless plates (11a, 11b) in a stainless steel vessel keeping a membrane between them, which plates are equipped with rectangular spiral grooves arranged to enable overlapping contact between the sample solution on one side of the membrane and the carrier gas and the other side of the membrane.

17. An instrument according to claim 16, **characterized in that** the width of the spiral grooves is 2 mm and the depth is 1 mm.

18. An instrument according to any one of claims 13 to 17, **characterized in that** the membrane is a microporous membrane allowing the analyte to pass from the sample solution to the carrier gas.

19. An instrument according to any one of claim 18, **characterized in that** the microporous membrane is a polypropylene membrane having an effective area of 3 cm².

20. An instrument according to claim 19, **characterized in that** the thickness of the membrane is 50 µm and the porosity is 45 %.

21. An instrument according to any one of claims 13 to 20, **characterized by** a water trap (9) between the membrane device (2) and the ion mobility spectrometer (1), which water trap (9) prevents humidity in the sample gas from entering into the ion mobility spectrometer (1).

22. An instrument according to any one of claims 13 to 21, **characterized in that** the ion mobility spectrometer (1) is adapted to measure the deviation of the signal measured at the single electrodes from a background signal and to compare the sample response thus obtained with a corresponding signal obtained with a calibration sample.

23. An instrument according to any one of claims 13 to 22, **characterized in that** it is adapted to measure the collected analyte ions continuously on-line.

## Patentansprüche

1. Verfahren zum Bestimmen des Gehalts an zu analysierender Substanz einer Probe aus einem Produktionsverfahren mit Hilfe von Ionenmobilitätsspektroskopie, wobei das Verfahren die folgenden Schritte umfasst:
Extrahieren der zu analysierenden Substanz aus der Probe mit Hilfe eines Gases und zum Zwecke, ein Probengas zu bilden, welches die zu analysierende Substanz enthält;
Ionisieren des Probengases;
Hindurchleiten des ionisierten Probengases durch ein Messrohr, welches Elektroden zum Erzeugen elektrischer Sammelfelder enthält, und welches im wesentlichen senkrecht zur Bewegungsrichtung des Probengases angeordnet ist;
Messen des elektrischen Stromes, der durch die an den besagten Elektroden aufgesammelten Ionen erzeugt wird, wobei der besagte Strom ein Maß ist für den Gehalt an zu analysierender Substanz;
**dadurch gekennzeichnet, dass**
die Probe als Probenlösung vom Produktionsverfahren an eine Membranöffnung geführt wird sowie entlang einer Seite der Membran; und dass ein Strom des Trägergases zur besagten Membranöffnung geleitet wird sowie entlang der anderen Seite der Membran,
wobei die zu analysierende Substanz von der Probenlösung durch die Membran in das Trägergas übergeht, und zwar wegen Diffusion und laminarer /turbulenter Strömungswechselwirkung, um das besagte Probengas zu bilden, welches die zu analysierende Substanz enthält.

2. Verfahren nach Anspruch 1, **dadurch gekennzeichnet, dass** das Trägergas Luft ist.

3. Verfahren nach Anspruch 1 oder 2, **dadurch gekennzeichnet, dass** der Gehalt an zu analysierender Substanz dadurch bestimmt wird, dass der elektrische Strom, der an den einzelnen Elektroden durch die Probe, die zu messen ist, erzeugt wird, jeweils verglichen wird mit dem Strom einer Kalibrierprobe.

4. Verfahren nach einem der Ansprüche 1, 2 oder 3, **dadurch gekennzeichnet, dass** das Sammeln von Ionen gemessen wird, wobei die Ionen unterschiedliche Mobilität in verschiedenen Elektroden aufweisen.

5. Verfahren nach mindestens einem der vorstehenden Ansprüche, **dadurch gekennzeichnet, dass** die Temperatur der Probenlösung geregelt wird.

6. Verfahren nach mindestens einem der vorstehenden Ansprüche, **dadurch gekennzeichnet, dass** das Messen der aufgesammelten Ionen der zu analysierenden Substanz kontinuierlich online durchgeführt wird.

7. Verfahren nach mindestens einem der vorstehenden Ansprüche, **dadurch gekennzeichnet, dass** die Probenlösung, die vom Produktionsverfahren kommt, über einen Wärmetauscher zur Membranöffnung gepumpt wird.

8. Verfahren nach mindestens einem der vorstehenden Ansprüche, **dadurch gekennzeichnet, dass** das Probengas durch eine Wasserfalle geleitet wird bevor es zum Zwecke der Ionenmobilitätsspektroskopie ionisiert wird.

9. Verfahren nach mindestens einem der vorstehenden Ansprüche, **dadurch gekennzeichnet, dass** die Flussrate des Probengases zum Ionenmobilitätsspektrometer von 1 bis 3 l/min, reicht, bevorzugt von 2 bis 2,5 l/min.

10. Verfahren nach mindestens einem der Ansprüche 1 bis 7 und Anspruch 9, **dadurch gekennzeichnet, dass** das Probengas direkt zum Ionenmobilitätsspektrometer geleitet wird.

11. Verfahren nach mindestens einem der vorstehenden Ansprüche, **dadurch gekennzeichnet, dass** das Produktionsverfahren ein biologisches Produktionsverfahren ist, vorzugsweise eine Fermentierungsreaktion, eine Fermentierung von Hefe oder Bakterien und/oder dass die zu analysierende Substanz Ethanol ist oder ein anderes flüchtiges Produkt aus einem biologischen Produktionsverfahren.

12. Verfahren nach mindestens einem der vorstehenden Ansprüche, **dadurch gekennzeichnet, dass** das ionisierte Probengas aufgeteilt wird in zwei im wesentlichen gleiche Anteile, für welche dann der Gehalt an zu analysierender Substanz gemessen wird.

13. Vorrichtung zum Messen des Gehaltes an einer zu analysierenden Substanz einer Probe aus einem Produktionsverfahren, umfassend ein Reaktionsgefäß (3) sowie ein Ionenmobilitätsspektrometer (1) zum Messen des Gehalts an zu analysierender Substanz, **dadurch gekennzeichnet, dass** diese Vorrichtung weiterhin umfasst: eine Pumpe für eine flüssige Probe (5), eine Einrichtung mit Membran (2) mit einer Membran, die den Durchtritt von Molekülen der zu analysierenden Substanz von der flüssigen in die Gasphase erlaubt, sowie eine Gaspumpe (4), wobei diese so angeordnet sind, dass die Pumpe für die flüssige Phase (5) die Probe als Probenlösung vom Reaktionsgefäß (3) auf eine Seite der Membran pumpt, sowie dass die Gaspumpe (4) Probengas auf die andere Seite der Membran pumpt, wobei die zu analysierende Substanz von der Probenlösung durch die Membran in das Probengas durchtritt, und zwar bedingt durch Diffusion und laminare /turbulente Strömungswechselwirkung, um ein Probengas zu bilden, welches die zu analysierende Substanz enthält, und wobei die Pumpe weiterhin das Probengas zum Ionenmobilitätsspektrometer (1) pumpt.

14. Vorrichtung gemäß Anspruch 13, **dadurch gekennzeichnet, dass** das Trägergas Luft ist.

15. Vorrichtung gemäß Anspruch 13 oder 14, **dadurch gekennzeichnet, dass** ein Wärmetauscher (7) zwischen der Pumpe für die flüssige Probe (5) und der Einrichtung mit Membran (2) so eingerichtet ist, dass die Probenlösung aufgeheizt wird, bevor sie die Membran erreicht.

16. Vorrichtung nach einem der Ansprüche 12, 13 oder 14, **dadurch gekennzeichnet, dass** die Einrichtung mit Membran (2) eine bilobale Einrichtung mit Membran ist, umfassend scheibenförmige Platten aus Edelstahl (11a, 11b) in einem Reaktionsgefäß aus Edelstahl, wobei eine Membran zwischen diesen angebracht ist, und wobei die besagten Platten ausgerüstet sind mit rechteckig spiralförmigen Rinnen, die so angeordnet sind, dass sie einen überlappenden Kontakt zwischen der Probenlösung auf der einen Seite der Membran und dem Probengas auf der anderen Seite der Membran ermöglichen.

17. Vorrichtung gemäß Anspruch 16, **dadurch gekennzeichnet, dass** die Breite der spiralförmigen Rillen 2 mm ist und die Tiefe 1 mm.

18. Vorrichtung gemäß mindestens eines der Ansprüche 13 bis 17, **dadurch gekennzeichnet, dass** die Membran eine mikroporöse Membran ist, die es erlaubt, dass die zu analysierende Substanz von der Probenlösung in das Trägergas durchtreten kann.

19. Vorrichtung gemäß Anspruch 18, **dadurch gekennzeichnet, dass** die mikroporöse Membran eine Polypropylen Membran ist mit einer effektiven Fläche von 3 cm².

20. Vorrichtung gemäß Anspruch 19, **dadurch gekennzeichnet, dass** die Dicke der Membran 50 um beträgt sowie die Porosität 45 %

21. Vorrichtung gemäß mindestens eines der Ansprüche 13 bis 20, **dadurch gekennzeichnet, dass** sich eine Wasserfalle (9) zwischen der Einrichtung mit Membran (2) und dem Ionenmobilitätsspektrometer (1) befindet, wobei die Wasserfalle (9) verhindert, dass Feuchtigkeit vom Probengas in das Ionenmobilitätsspektrometer übertritt.

22. Vorrichtung gemäß mindestens eines der Ansprüche 13 bis 21, **dadurch gekennzeichnet, dass** das Ionenmobiltätsspektrometer (1) so angepasst ist, dass der Unterschied des an den Einzelelektroden gemessenen Signals von einem Hintergrundsignal zu messen, sowie die so erhaltene, durch die Probe induzierte, Antwort zu vergleichen mit einem entsprechenden Signal von einer Probe zur Kalibrierung.

23. Vorrichtung gemäß mindestens eines der Ansprüche 13 bis 22, **dadurch gekennzeichnet, dass** besagte Vorrichtung dazu geeignet ist, die gesammelten Ionen der zu analysierenden Substanz kontinuierlich und online zu messen.

## Revendications

1. Procédé pour déterminer, par spectrométrie à mobilité d'ions, la proportion d'analyte d'un échantillon de processus de fabrication, comprenant les étapes consistant à :
extraire l'analyte de l'échantillon au moyen d'un gaz afin de former un échantillon gazeux qui contient l'analyte ;
ioniser l'échantillon gazeux ;
conduire l'échantillon gazeux ionisé à travers un tube de mesure comprenant des électrodes pour générer des champs électriques de collecte essentiellement verticaux par rapport à la direction du déplacement de l'échantillon gazeux ;
mesurer le courant électrique généré par les ions collectés dans lesdites électrodes, ledit courant étant une mesure de la proportion d'analyte ;
**caractérisé en ce que** :
l'on conduit un échantillon sous la forme d'une solution d'échantillon à partir du processus de fabrication vers une entrée de membrane et le long d'une face de la membrane ; et
l'on conduit un courant du gaz porteur vers ladite entrée de membrane et le long de l'autre face de la membrane,
grâce à quoi l'analyte passe de la solution d'échantillon, par diffusion et interaction de flux laminaire/turbulent, à travers la membrane jusqu'à l'intérieur du gaz porteur pour former ledit échantillon gazeux qui contient l'analyte.

2. Procédé selon la revendication 1, **caractérisé en ce que** le gaz porteur est de l'air.

3. Procédé selon la revendication 1 ou 2, **caractérisé en ce que** la proportion d'analyte est dérivée en comparant le courant électrique produit au niveau des électrodes individuelles par l'échantillon devant être mesuré et par un échantillon d'étalonnage, respectivement.

4. Procédé selon la revendication 1, 2 ou 3, **caractérisé en ce que** la collecte d'ions ayant une mobilité différente dans des électrodes différentes est mesurée.

5. Procédé selon l'une quelconque des revendications précédentes, **caractérisé en ce que** la température de la solution d'échantillon est régulée.

6. Procédé selon l'une quelconque des revendications précédentes, **caractérisé en ce que** la mesure des ions d'analyte collectés est effectuée de façon continue en ligne.

7. Procédé selon l'une quelconque des revendications précédentes, **caractérisé en ce que** la solution d'échantillon est pompée à partir du processus de fabrication par l'intermédiaire d'un échangeur de chaleur vers l'entrée de membrane.

8. Procédé selon l'une quelconque des revendications précédentes, **caractérisé en ce que** l'échantillon gazeux est amené à travers un piège à eau avant qu'il ne soit ionisé pour la spectrométrie à mobilité d'ions.

9. Procédé selon l'une quelconque des revendications précédentes, **caractérisé en ce que** le débit d'écoulement de l'échantillon gazeux vers le spectromètre à mobilité d'ions est compris entre 1 et 3 l/min, et, de préférence, entre 2 et 2,5 l/min.

10. Procédé selon l'une quelconque des revendications 1 à 7 et 9, **caractérisé en ce que** l'échantillon gazeux est amené directement vers le spectromètre à mobilité d'ions.

11. Procédé selon l'une quelconque des revendications précédentes, **caractérisé en ce que** le processus est un processus biologique, de préférence une réaction de fermentation, une fermentation par levures ou bactéries et/ou **en ce que** l'analyte est de l'éthanol ou un autre produit de processus biologique volatile.

12. Procédé selon l'une quelconque des revendications précédentes, **caractérisé en ce que** l'échantillon gazeux ionisé est divisé en deux flux essentiellement similaires, à partir desquels la proportion d'analyte est mesurée.

13. Instrument pour mesurer une proportion d'analyte d'un échantillon d'un processus de fabrication, comprenant une cuve de réaction (3) et un spectromètre à mobilité d'ions (1) pour mesurer la proportion d'analyte, **caractérisé en ce qu'**il comprend de plus une pompe d'échantillon liquide (5), un dispositif à membrane (2) comportant une membrane permettant la perméation des molécules d'analyte d'une phase liquide à une phase gazeuse, et une pompe à gaz (4), qui sont disposés de telle sorte que la pompe d'échantillon liquide (5) pompe l'échantillon sous la forme d'une solution d'échantillon à partir de la cuve (3) vers une face de la membrane, la pompe à gaz (4) pompe le gaz porteur vers l'autre face de la membrane, grâce à quoi l'analyte passe de la solution d'échantillon, grâce à la diffusion et à l'interaction de flux laminaire/turbulent à travers la membrane, jusqu'à l'intérieur du gaz porteur de façon à former un échantillon gazeux contenant l'analyte, et pompe de plus l'échantillon gazeux vers le spectromètre à mobilité d'ions (1).

14. Instrument selon la revendication 13, **caractérisé en ce que** le gaz porteur est de l'air.

15. Instrument selon la revendication 13 ou 14, **caractérisé en ce qu'**un échangeur de chaleur (7) est disposé entre la pompe d'échantillon liquide (5) et le dispositif à membrane (2) pour chauffer la solution d'échantillon avant qu'elle n'atteigne la membrane.

16. Instrument selon la revendication 12, 13 ou 14, **caractérisé** en ce le dispositif à membrane (2) est un dispositif à membrane bilobée, comprenant des plaques inoxydables discoïdales (11a, 11b) dans une cuve en acier inoxydable maintenant une membrane entre elles, ces plaques étant équipées de rainures spirales rectangulaires configurées de façon à permettre un contact de chevauchement entre la solution d'échantillon d'un côté de la membrane et le gaz porteur de l'autre côté de la membrane.

17. Instrument selon la revendication 16, **caractérisé** en ce la largeur des rainures spirales est de 2 mm et en ce que la profondeur est de 1 mm.

18. Instrument selon l'une quelconque des revendications 13 à 17, **caractérisé en ce que** la membrane est une membrane microporeuse, permettant à l'analyte de passer de la solution d'échantillon au gaz porteur.

19. Instrument selon la revendication 18, **caractérisé en ce que** la membrane microporeuse est une membrane de polypropylène ayant une surface efficace de 3 cm².

20. Instrument selon la revendication 19, **caractérisé en ce que** l'épaisseur de la membrane est de 50 µm et **en ce que** la porosité est de 45 %.

21. Instrument selon l'une quelconque des revendications 13 à 20, **caractérisé par** un piège à eau (9) entre le dispositif de membrane (2) et le spectromètre à mobilité d'ions (1), ce piège à eau (9) empêchant l'humidité dans l'échantillon gazeux d'entrer à l'intérieur du spectromètre à mobilité d'ions (1).

22. Instrument selon l'une quelconque des revendications 13 à 21, **caractérisé en ce que** le spectromètre à mobilité d'ions (1) est adapté pour mesurer l'écart du signal mesuré au niveau des électrodes individuelles par rapport à un signal d'arrière-plan et pour comparer la réponse de l'échantillon ainsi obtenue à un signal correspondant obtenu avec un échantillon d'étalonnage.

23. Instrument selon l'une quelconque des revendications 13 à 22, **caractérisé en ce qu'**il est adapté pour mesurer les ions d'analyte collectés de façon continue en ligne.
